(19)

**Europäisches Patentamt**

**European Patent Office**

**Office européen des brevets**

(11) **EP 3 114 477 B1**

(12) **EUROPEAN PATENT SPECIFICATION**

(45) Date of publication and mention
of the grant of the patent:
**26.12.2018 Bulletin 2018/52**

(21) Application number: **15716866.7**

(22) Date of filing: **04.03.2015**

(51) Int Cl.:
**G01N 33/38** *(2006.01)*      **G01N 27/403** *(2006.01)*

(86) International application number:
**PCT/IB2015/051580**

(87) International publication number:
**WO 2015/132737 (11.09.2015 Gazette 2015/36)**

(54) **SYSTEM AND METHOD FOR DETECTING AND MONITORING CHLORINE ION CONCENTRATION AND PH IN CONCRETE STRUCTURES**

SYSTEM UND VERFAHREN ZUR DETEKTION UND ÜBERWACHUNG DER CHLOR-IONENKONZENTRATION UND DES PH-WERTS IN BETONSTRUKTUREN

SYSTÈME ET PROCÉDÉ DE DÉTECTION ET DE SURVEILLANCE DE CONCENTRATION EN IONS DE CHLORE ET DU PH DANS DES STRUCTURES EN BÉTON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.03.2014  IT PA20140004**

(43) Date of publication of application:
**11.01.2017  Bulletin 2017/02**

(73) Proprietor: **TEM LAB S.R.L.**
**90044 Carini (PA) (IT)**

(72) Inventors:
 • **ALESSI, Aldo**
  **I-90100 Palermo (PA) (IT)**
 • **CARCARA, Filippo**
  **I-91028 Partanna (TP) (IT)**
 • **CERAULO, Manuela**
  **I-90100 Palermo (PA) (IT)**
 • **CINA', Gaetano**
  **I-90100 Palermo (PA) (IT)**
 • **DE LUCA, Salvatore**
  **I-90100 Palermo (PA) (IT)**
 • **GIANNICI, Francesco**
  **I-90100 Palermo (PA) (IT)**
 • **GIARRUSSO, Renato**
  **I-90100 Palermo (PA) (IT)**
 • **INGUANTA, Rosalinda**
  **I-90028 Palma di Montechiaro (AG) (IT)**

 • **LA MANTIA, Francesco Paolo**
  **I-90100 Palermo (PA) (IT)**
 • **MISTRETTA, Maria Chiara**
  **I-90100 Palermo (PA) (IT)**
 • **MULONE, Angelo**
  **I-90139 Palermo (PA) (IT)**
 • **MULONE, Antonio**
  **I-90139 Palermo (PA) (IT)**
 • **PUSATERI, Michele**
  **I-90127 Palermo (PA) (IT)**
 • **TERRANOVA, Luigi Maria**
  **I-90100 Palermo (PA) (IT)**
 • **TRAMAGLINO, Calogero**
  **I-90049 Terrasini (PA) (IT)**

(74) Representative: **Maroscia, Antonio**
**Maroscia & Associati Srl**
**Piazza del Castello, 26**
**36100 Vicenza (IT)**

(56) References cited:
**WO-A1-2007/109560    US-A1- 2008 179 179**

 • **RONG-GUI DU ET AL: "In Situ Measurement of Cl - Concentrations and pH at the Reinforcing Steel/Concrete Interface by Combination Sensors", ANALYTICAL CHEMISTRY, vol. 78, no. 9, 1 May 2006 (2006-05-01), pages 3179-3185, XP055148488, ISSN: 0003-2700, DOI: 10.1021/ac0517139 cited in the application**

**Description**

Field of the Invention

The present invention relates to a system for detecting and monitoring chloride ion concentration and pH in concrete structures.

The invention also relates to a method for detecting and monitoring chloride ion concentration and pH in such structures.

Background art

In the field of building, concrete degradation caused by the presence of chloride ions and pH reduction at the interface with the metallic reinforcement in concrete is known to be monitored.

Systems have been developed for monitoring chloride ion concentration and pH, which are based on core sampling, high-pressure concrete extraction, or acid/water concrete extraction.

A first drawback of these processes is that they are highly invasive and necessarily involve destruction of the concrete sample being tested.

As a result these processes are hardly implemented on-site and cannot be continuously iterated, therefore they cannot provide real-time concentration values.

A further drawback is that the equipment for these processes is particularly expensive and do not allow quick testing of concrete structures.

In view of at least partially obviating these drawbacks, methods and systems have been developed, which allow quick, non-destructive chloride ion and pH detection by detection and processing of the redox potentials of the chemical species being monitored in concrete structures.

A system for on-site concrete structure monitoring is known from RONG-GUI DU ET AL: "In Situ Measurement of CI - Concentrations and pH at the Reinforcing Steel/Concrete Interface by Combination Sensors", ANALYTICAL CHEMISTRY, vol. 78, no. 9, 1 May 2006 (2006-05-01), pages 3179-3185, ISSN: 0003-2700, DOI: 10.1021/ac0517139, which uses a device comprising Ag/AgCI and Ir/IrO$_2$ electrodes for detecting chloride ions and pH.

The electrodes are placed in respective housings previously formed in the concrete structure, a counter-electrode being also placed in a hole formed in the structure and buried in a conductive liquid. The detected potential values are later used to calculate chloride ion concentration and pH using the known Nernst formula.

A limit of this device is that it provides excessively approximate chloride ion concentration and pH measurements, as it does not account for all input variables of the Nernst formula.

Therefore, the use of such device is limited to very stable and controlled temperature conditions, to avoid possibly considerable alterations of the electric quantities monitored by the electrodes.

A further drawback of this known system is that the level of conductive liquid in contact with the counter-electrode decreases with time due to normal leakage occurring in the structure, and this may considerably alter ion concentration and pH measurements.

This drawback may cause oxidation of the counter-electrode and inevitably lead with time to its degradation.

US2008179179 A1 and WO2007109560 A1 disclose systems for measuring, respectively, an oxidation-reduction potential and pH and both comprising temperature detectors for compensating and correcting the measurements with temperature data.

A limit of these systems is that they cannot be used in combination with concrete structures and if they would be, they wouldn't have electric continuity with that and they should be removed to be washed.

Disclosure of the invention

A general object of the present invention is to obviate the above drawbacks, by providing a method and a system for detecting and monitoring chloride ions and pH in concrete structures, that are highly efficient and relatively cost-effective.

A particular object of the present invention is to provide a detection and monitoring method that can be used on-site and provide very accurate real-time results.

A particular object of the present invention is to provide a detection and monitoring method that can be used on-site and provide very accurate real-time results.

_A particular object of the present invention is to provide a detection and monitoring method that do not involve destruction of the sample being tested.

Another object of the present invention is to provide a detection and monitoring method and system that provide real-time concentration values with high accuracy and sensitivity.

A further object of the present invention is to provide an automatic system for detecting and monitoring chloride ion concentration and pH.

The above mentioned objects, as well as others to be better explained hereafter, are fulfilled by a system for detecting and monitoring chloride ions and pH in concrete structures as defined in claim 1 and a method of detecting and monitoring chloride ions and pH in concrete structures as defined in claim 6.

Advantageous embodiments of the invention are obtained in accordance with the dependent claims.

Brief Description of the Drawings

Further features and advantages of the invention will be more apparent from the detailed description of a preferred, non-exclusive embodiment of a system and method for detecting and monitoring chloride ion concentration and pH in concrete structures according to the invention, which are described as a non-limiting example with the help of the annexed drawings, in which:

FIG. 1 is a first general schematic view of the detection system of the invention, installed in a concrete structure to be monitored;
FIG. 2 is a first enlarged detail of Fig. 3;
FIG. 3 is a second enlarged detail of Fig. 1;
FIG. 4 is an implementation diagram of the system of Fig. 1;

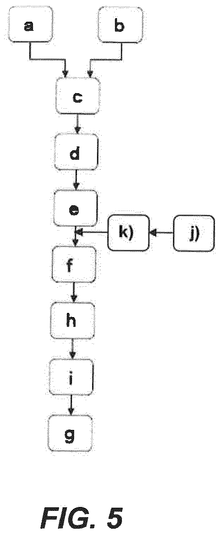

Fig. 5 is a block diagram of the chloride ion concentration and pH detection method of the invention.

Detailed description of a preferred embodiment

Referring to the above figures, there is shown a system for detecting and monitoring chloride ion concentration and pH, which can be used with a building structure S made of reinforced concrete or structural concrete, possibly comprising lithoidal materials or concrete mixes, generally designated by numeral 1 .

With the detection of chloride ion concentration and pH, concrete reinforcement corrosion may be monitored and prevented, and the carbonation degree of the material may be measured.

The system 1 may be used both on consolidated concrete structures and on relatively fresh concrete castings for new structures.

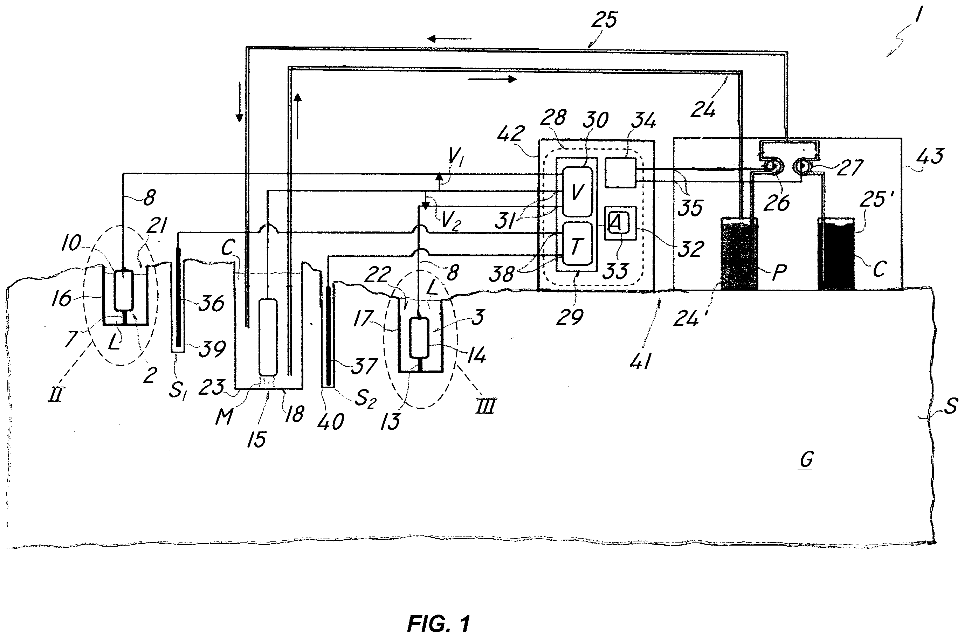

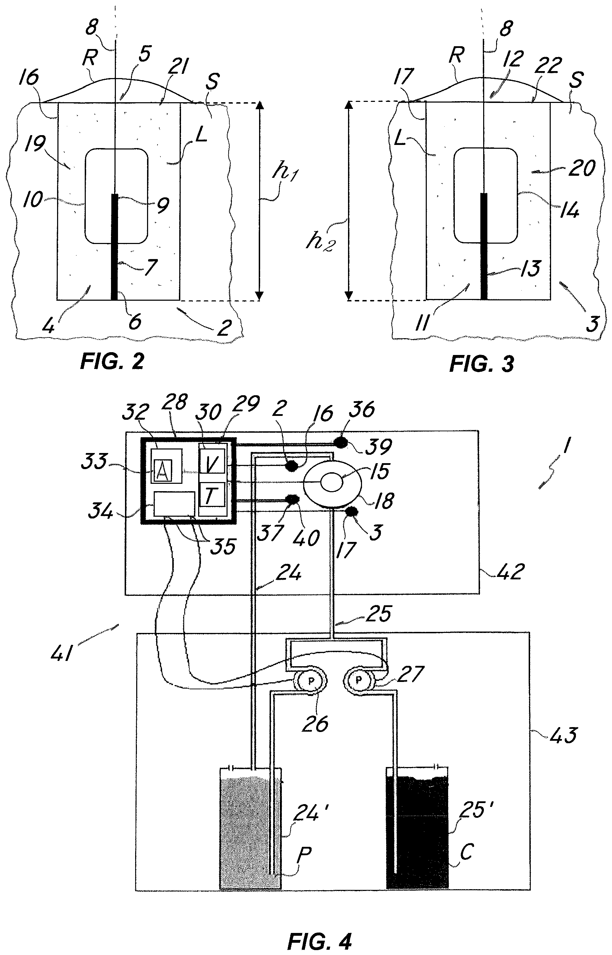

As shown in FIGS. 1 and 2, the system 1 of the present invention comprises at least one first electrode 2, embedded in the structure S, for detecting chloride ions and at least one second electrode 3, also buried in the structure S, for detecting the pH.

A first electrode 2 may have an active Ag/AgCl part, which is designed to contact the structure S, and a conductive part 5, which is electrically connected to the active part 4 and projects from the structure S.

The active part 4 may be formed by an anodization process using hydrochloric acid HCl as the electrolyte solution.

In order to obtain a compact and uniform Ag/AgCl layer, anodization will be carried out on a platinum gauze, not shown, which is wrapped around one end 6 of a silver wire 7 having a length close to about 1 cm.

The conductive part 5 may be formed as a copper braid 8, which is joined to the opposite end 9 of the silver wire 7.

The copper braid 8 may have a length of about 10 cm and will be covered by a rubber or plastic sheath, not shown, for electric insulation.

Conveniently, the first electrode 2 will be covered by a layer 10 of a two-component insulating resin at the connection between the copper braid 8 and the end 9 of the silver wire 7.

The second electrode 3 may also comprise an active $Ir/IrO_2$ part, designed to contact the structure S, and a conductive part 12 projecting from the structure S.

Here, the active part 11 of the second electrode 3 may be formed by immersing an iridium wire 13 in calcium carbonate heated to a temperature of about 700°C in a muffle for about 60 minutes.

The iridium wire 13 may have a length of about 1 cm and once again the conductive part 12 of the second electrode 3 may be formed as a copper braid 8, having a length of about 20 cm.

The second conductive part 12 may be electrically insulated by covering the second electrode 3 with an epoxy resin.

The system 1 also comprises a metal counter-electrode 15 which can be located in contact with the structure S proximate to the first electrode 2 and the second electrode 3.

The first electrode 2, the second electrode 3 and the counter-electrode 15 are adapted to be held within respective housing holes 16, 17, 18 formed in the structure S to be monitored.

Particularly, the housing holes 16, 17 of the first electrode 2 and the second electrode 3 may have a small diameter, e.g. about 5mm, and may have equal or different depths $h_1$, $h_2$ depending on whether monitoring has to be performed on a particular area of the concrete or throughout the thickness of the casting G.

Furthermore, the housing holes 16, 17 of the first electrode 2 and the second electrode 3 may be located at substantially the same distance from the housing hole 18 of the counter-electrode 15.

Conveniently, once the first electrode 2 and the second electrode 3 have been introduced into their respective housing, the cavities 19, 20 of the holes 16, 17 may be filled with liquid plaster L.

The outer edges 21, 22 of the holes 16, 17 will be preferably covered with a sealing resin R to waterproof the electrodes 2, 3.

The counter-electrode 15 will be also introduced into a corresponding housing hole 18 and will be bonded to the structure as by an appropriate rubber cement M distributed on the bottom wall 23 of the hole 18.

The system 1 further comprises a cleaning circuit 24 for cleaning the housing hole 18 of the counter-electrode 15, in which a cleaning liquid P is pumped to remove drilling debris from the hole 18.

Furthermore, an appropriate feeding circuit 25 is provided in which a conductive liquid C is pumped for at least partially drowning the counter-electrode 15 when the latter is introduced into the housing hole 18 that was previously cleared with the washing liquid P.

As the hole 18 is filled with the conductive liquid C, constant and uniform electric continuity will be ensured between the counter-electrode 15 and the structure S to be monitored.

The cleaning circuit 24 and the feeding circuit 25 may comprise respective metering pumps 26, 27, which are adapted to promote controlled feeding of the cleaning liquid P or the conductive liquid C from respective reservoirs 24', 25' into the hole 18 that houses the counter-electrode 15.

The system 1 further has microprocessor means 28, or data logger, comprising a measuring unit 29 for detecting electric voltages $V_1$, $V_2$ between each first electrode 2 and the counter-electrode 15, and between each second electrode 3 and the counter-electrode 15.

The measuring unit 29 may comprise a digital voltmeter 30 having first inputs 31 connected with the electrodes 2, 3 and with the counter-electrode 15 for detecting real-time values of such electric voltages $V_1$, $V_2$.

The microprocessor means 28 also comprise a processing unit 32, which is adapted to calculate the chloride ion concentration and pH values from the electric voltage values $V_1$, $V_2$ that have been measured by the measuring unit 29.

Particularly, the microprocessor means 28 may comprise a storage area 33 containing a computer program A having a series of instructions for calculating chloride ion concentration and pH using the known Nernst formula, i.e.:

$$V_x = V_0 + (RT_x/nF) \ln (C_{ox} / C_{red})$$

where $V_x$ is the electric voltage as measured between the first electrode 2 or the second electrode 3 and the counter-electrode 15, $V_0$ is the standard reduction potential, Tx is the Kelvin temperature, $C_{ox}$ and $C_{red}$ are the concentrations of the oxidized and reduced species of chloride ions or hydrogen ions respectively.

Preferably, the microprocessor means 28 may comprise a control unit 34 having outputs 35 connected to the metering pumps 26, 27 in the cleaning circuit 24 and the conductive liquid C feeding circuit 25 for controlling actuation and batch operation thereof.

Thus, the hole 18 that houses the counter-electrode 15 may be automatically cleaned and later filled with the conductive liquid C at regular intervals since installation of the system 1 in the structure S.

Furthermore, the processor means 28 may comprise cable or radio communication means, and GSM location means for transmitting and receiving data from a remote station designed for multiple systems installed on various structures, not shown.

The system 1 may possibly comprise level sensors, also not shown, which are connected to the electronic control unit 34 and are adapted to detect the amount of conductive liquid C in the hole 18 that houses the counter-electrode 15.

When the level of the conductive liquid C is lower than a predetermined threshold value, the control unit 34 will actuate the corresponding metering pump 27 to restore the level of the conductive liquid C.

The system 1 comprises at least one pair of thermocouples 36, 37.

The first thermocouple 36 is at a first location $S_1$ of the structure S near the first electrode 2 and/or the second electrode 3.

The second thermocouple 37 is at a second location $S_2$ in contact with the structure S proximate to the counter-electrode 15.

The thermocouples 36, 37 will be connected to second inputs 38 of the measuring unit 29, for detecting corresponding instantaneous temperatures $T_1$, $T_2$ at the first location $S_1$ and the second location $S_2$.

As best shown in FIG. 1, the first location $S_1$ and the second location $S_2$ may be provided by forming respective holes 39, 40 in the structure S for housing the thermocouples 36, 37.

The thermocouples 36, 37 are connected to the microprocessor means 28 such that the computer program A installed in the storage area 33 of the control unit 34 may calculate chloride ion concentration and pH values using the above Nernst formula, in which the temperature value $T_x$ is replaced by the instantaneous value $T_1$, $T_2$ as measured by the thermocouples 36, 37.

Conveniently, a kit 41 may be provided, comprising a first container 42 and a second container 43, which contain all the components of the system 1 of the invention.

Particularly, the first container 42 may contain microprocessor means 28, the electrodes 2, 3 and the counter-electrode 15, whereas the second container 43 will be designed to accommodate the reservoirs 24', 25' and the metering pumps 26, 27 associated with the cleaning circuit 24 and the feeding circuit 25.

The method of operation of the device is shown in the diagram of FIG. 3 and comprises steps for detection and monitoring of chloride ion concentration and pH in concrete structures.

The method comprises a step of a) providing at least one first electrode 2 for detecting chloride ions in a structure S and a step of b) providing at least one second electrode 3 for detecting pH as described above.

In step c) the first electrode 2 and the second electrode 3 are embedded in the concrete structure S before or after concrete casting G.

The first electrode 2 and the second electrode 3 are introduced into respective housing holes 16, 17 formed at different locations in the structure S. Furthermore, these housing holes 16, 17 may have equal or different depths $h_1$, $h_2$.

The next steps are d) providing a metal counter-electrode 15 and e) positioning the counter-electrode 15 in contact with the structure S near the first electrode 2 and second electrode 3.

The counter-electrode 15 is also inserted in a respective housing hole 18 formed in the structure S, which is later filled with a conductive liquid C to ensure constant electric continuity between the counter-electrode 15 and the structure S to be monitored.

The next step is f) measuring the electric voltages $V_1$, $V_2$ between the counter-electrode 15 and the first electrode 2 and the second electrode 3 respectively, preferably using a digital voltmeter 30.

The next step is g) calculating the chloride ion concentration and pH values from such measured electric voltages $V_1$, $V_2$. Such calculation will be made by the microprocessor means 28 and will be preceded by a step of h) positioning at least two thermocouples 36, 37, one of which at a first location $S_1$ in the structure S near the first electrode 2 and/or the second electrode 3, and the other at a second location $S_2$ in contact with the structure S proximate to the counter-electrode 15.

In step i) respective instantaneous temperatures $T_1$, $T_2$ will be detected by the thermocouples 36, 37 such that the calculated concentrations may account for these measured temperature values $T_1$, $T_2$.

Preferably, the microprocessor means 28 will be configured to repeat the calculation step g) at predetermined time intervals to determine a time curve of chloride ion concentration and pH in the structure.

The microprocessor means 28 will perform the calculation step g) using the Nernst formula in which the variables $V_x$ and $T_x$ are replaced by the corresponding electric potentials $V_1$, $V_2$ and the instantaneous temperatures $T_1$, $T_2$ as detected by the voltmeter 30 and the thermocouples 36, 37 respectively.

While the method and system have been described with particular reference to the accompanying figures, the numerals referred to in the disclosure and claims are only used for the sake of a better intelligibility of the invention and shall not be intended to limit the claimed scope in any manner.

**Claims**

1. A system for detecting and monitoring chloride ion concentration and pH in a concrete structure (S), comprising:

   - at least one first electrode (2) for chloride ion detection designed to be inserted in a respective housing hole (16) of the concrete structure (S);
   - at least one second electrode (3) for pH detection designed to be inserted in a respective housing hole (17) of the concrete structure (S);
   - a counter-electrode (15) designed to be inserted in a respective housing hole (18) of the concrete structure (S) in contact therewith;
   - microprocessor means (28) comprising a measuring unit (29) for measuring electric voltages ($V_1$, $V_2$) between said counter-electrode (15) and said at least one first electrode (2) or said at least one second electrode (3), and a processing unit (32);

   **characterised in that** the system comprises at least two thermocouples (36, 37), one of which is adapted to be at a first location ($S_1$) of the concrete structure (S) proximate to said at least one first electrode (2) and/or second electrode (3) and the other is adapted to be at a second location ($S_2$) in contact with the concrete structure (S) proximate to said counter-electrode (15) to measure respective instantaneous temperatures ($T_1$, $T_2$), said processing unit (32) being connected to said thermocouples (36, 37) and adapted for calculating chloride ion concentration and pH from the measured values of said electric voltages ($V_1$, $V_2$) and from said instantaneous temperature values ($T_1$, $T_2$), and **in that** the system further comprises a cleaning circuit (24) for delivering a cleaning liquid (P) and a feeding circuit (25) for supplying a conductive liquid (C) to the housing hole (18) for said counter-electrode (15).

2. A system as claimed in claim 1, **characterised in that** said at least one first electrode (2) has a first Ag/AgCl active part (4) adapted to be located within the concrete structure (S) and a first copper conductive part (5) adapted to project out of the concrete structure (S).

3. A system as claimed in claim 1, **characterised in that** said at least one second electrode (3) has a second Ir/IrO$_2$ active part (11) adapted to be located within the concrete structure (S) and a second copper conductive part (12) adapted to project out of the concrete structure (S).

4. A system as claimed in claim 1, **characterised in that** said processing unit (32) comprises a storage area (33) having a computer program (A) installed therein, which comprises a series of instructions for calculating chloride ion concentration and pH using an appropriate algorithm corresponding to the Nernst formula.

5. A system as claimed in claim 1, **characterised in that** each of said circuits (24, 25) comprises a respective metering pump (26, 27), said microprocessor means (28) comprising an electronic control unit (34) connected to each of the metering pumps (26, 27) for controlling the supply of said cleaning liquid (P) and/or said conductive liquid (C) into said housing hole (18) for said counter-electrode (15).

6. A method of detecting and monitoring chloride concentration and pH in a concrete structure using the system as claimed in one of the preceding claims, which method comprises the steps of:

   a) providing at least one first electrode (2) for chloride ion detection in the concrete structure (S) and inserting it in a respective housing hole (16) of the concrete structure (S);
   b) providing at least one second electrode (3) for pH detection in the concrete structure (S) and inserting it in a respective housing hole (17) of the concrete structure (S);

c) embedding said at least one first electrode (2) and second electrode (3) in the concrete structure (S) before or after concrete casting (G);

d) providing a counter-electrode (15);

e) positioning said counter-electrode (15) in contact with the structure (S) near said at least one first electrode (2) and said at least one second electrode (3) and inserting it in a respective housing hole (18) of the concrete structure (S);

f) measuring electric voltages ($V_1$, $V_2$) between said counter-electrode (15) and said at least one first electrode (2) and said at least one second electrode (3);

g) calculating the chloride ion concentration and pH values by microprocessor means (28);

**characterised in that**, said calculation step g) is preceded by a step of h) positioning at least two thermocouples (36, 37) one of which is at a first location ($S_1$) of the concrete structure (S) proximate to said at least one first electrode (2) and/or said at least one second electrode (3) and the other is at a second location ($S_2$) in contact with the concrete structure (S) proximate to said counter-electrode (15), a step being provided of i) detecting respective instantaneous temperatures ($T_1$, $T_2$) by said thermocouples (36, 37), said calculation step g) being carried out based on said measured electric voltages ($V_1$, $V_2$) and said instantaneous temperature values ($T_1$, $T_2$) as measured by said thermocouples (36, 37), said measuring step f) being preceded by a step j) of filling the housing hole (18) for the counter-electrode (15) with a cleaning liquid (P) and a step k) of filling the housing hole (18) for the counter electrode (15) with a conductive liquid (C).

7. A method as claimed in claim 6, **characterised in that** said step of g) calculating chloride ion concentration and pH is carried out using an algorithm corresponding to the Nernst equation.

8. A method as claimed in claim 6, **characterised in that** said calculation step g) is repeated at predetermined time intervals to determine a time curve of chloride ion concentration and pH in the concrete structure (S).

**Patentansprüche**

1. Ein System zur Ermittlung und Überwachung der Konzentration von Chlorid-Ionen und pH-Wert in einem Bauwerk aus Beton (S), umfassend:

- mindestens eine erste Elektrode (2) zur Ermittlung der Chlorid-Ionen, die in eine entsprechende Aufnahmeöffnung (16) des Bauwerks aus Beton (S) eingeführt wird;

- mindestens eine zweite Elektrode (3) zur Ermittlung des pH-Wertes, die in eine entsprechende Aufnahmeöffnung (17) des Bauwerks aus Beton (S) eingeführt wird;

- eine Gegenelektrode (15), die in eine entsprechende Aufnahmeöffnung (18) des Bauwerks aus Beton (S) eingeführt wird und sich im Kontakt zu diesem befindet;

- eine Mikroprozessoreinheit (28) einschließlich eines Messgeräts (29) zur Messung der elektrischen Spannungen ($V_1$, $V_2$) zwischen der genannten Gegenelektrode (15) und der genannten mindestens einen ersten Elektrode (2) oder der genannten mindestens einen zweiten Elektrode (3), und einer Verarbeitungseinheit (32);

**dadurch gekennzeichnet, dass** das System mindestens zwei Thermoelemente (36, 37) umfasst, von denen eines an einer ersten Position ($S_1$) des Bauwerks aus Beton (S) in der Nähe der genannten mindestens einen ersten Elektrode (2) und/oder zweiten Elektrode (3) angewendet wird und das zweite an einer zweiten Position ($S_2$) im Kontakt zum Bauwerk aus Beton (S) in der Nähe der genannten Gegenelektrode (15) angewendet wird, um die entsprechenden Istwerten der Temperatur ($T_1$, $T_2$) zu messen, und die genannte Verarbeitungseinheit (32) mit den genannten Thermoelementen (36, 37) verbunden ist und zur Berechnung der Konzentration der Chlorid-Ionen und des pH-Wertes aus den Messwerten der genannten elektrischen Spannungen ($V_1$, $V_2$) und aus den genannten Istwerten der Temperatur ($T_1$, $T_2$) verwendet wird, und dass das System außerdem einen Reinigungskreis (24) zur Beförderung einer Reinigungsflüssigkeit (P) und einen Zufuhrkreis (25) zur Zufuhr einer leitfähigen Flüssigkeit (C) zur Aufnahmeöffnung (18) für die genannte Gegenelektrode (15) umfasst.

2. Ein System gemäß Patentanspruch 1, **dadurch gekennzeichnet dass** die genannte mindestens eine Elektrode (2) einen ersten aktiven Teil Ag/AgCl (4), der im Bauwerk aus Beton (S) positioniert wird, und einen ersten leitfähigen Teil aus Kupfer (5) aufweist, der außerhalb des Bauwerks aus Beton (S) herausragt.

3. Ein System gemäß Patentanspruch 1, **dadurch gekennzeichnet dass** die genannte mindestens eine zweite Elektrode (3) einen zweiten aktiven Teil Ir/IrO$_2$ (11), der Innerhalb des Bauwerks aus Beton (S) positioniert wird, und einen zweiten leitfähigen Teil aus Kupfer (12) aufweist, der aus dem Bauwerk aus Beton (S) herausragt.

4. Ein System gemäß Patentanspruch 1, **dadurch gekennzeichnet dass** die genannte Verarbeitungseinheit (32) einen Speicherbereich (33) umfasst, der ein darin installiertes Computerprogramm (A) besitzt, das eine Reihe von Anweisungen zur Berechnung der Konzentration der Chlorid-Ionen und des pH-Wertes unter Verwendung eines dazu dienenden Algorithmus entsprechend der Nernst-Gleichung umfasst.

5. Ein System gemäß Patentanspruch 1, **dadurch gekennzeichnet dass** jeder der genannten Kreise (24, 25) eine jeweilige Dosierpumpe (26, 27) besitzt, wobei die genannten Mikroprozessormittel (28) eine elektronische Steuerungseinheit (34) umfassen, die jeweils mit jeder Dosierpumpe (26, 27) zur Steuerung der Einleitung der Reinigungsflüssigkeit (P) und/oder der genannten leitfähigen Flüssigkeit (C) in die genannte Aufnahmeöffnung (18) für die genannte Gegenelektrode (15) verbunden ist.

6. Ein Verfahren zur Ermittlung und Überwachung der Konzentration der Chlorid-Ionen und des pH-Wertes in einer Konstruktion aus Beton unter Verwendung des in einem der vorstehenden Patentansprüche beanspruchten Systems, dessen Methode folgende Schritte umfasst:

a) Bereitstellung mindestens einer ersten Elektrode (2) zur Ermittlung der Chlorid-Ionen im Bauwerk aus Beton (S) und deren Einführung in eine entsprechende Aufnahmeöffnung (16) des Bauwerks aus Beton (S);
b) Bereitstellung mindestens einer zweiten Elektrode (3) zur Ermittlung des pH-Wertes im Bauwerk aus Beton (S) und deren Einführung in eine entsprechende Aufnahmeöffnung (17) des Bauwerks aus Beton (S);
c) Einlegen der genannten ersten Elektrode (2) und zweiten Elektrode (3) in das Bauwerk aus Beton (S) vor oder nach dem Gießen des Betons (G);
d) Bereitstellung einer Gegenelektrode (15);
e) Positionierung der genannten Gegenelektrode (15) im Kontakt zum Bauwerk (S) in der Nähe von der genannten mindestens einen ersten Elektrode (2) und der genannten mindestens einen zweiten Elektrode (3) sowie deren Einführen in eine entsprechende Aufnahmeöffnung (18) des Bauwerks aus Beton (S);
f) Messen der elektrischen Spannungen ($V_1$, $V_2$) zwischen der genannten Gegenelektrode (15) und der genannten mindestens einen ersten Elektrode (2) und der genannten mindestens einen zweiten Elektrode (3);
g) Berechnung der Konzentration der Chlorid-Ionen und der pH-Werte mit der Mikroprozessoreinheit (28);

**dadurch gekennzeichnet dass** dem genannten Schritt zu Berechnung g) ein Schritt h) zur Positionierung von mindestens zwei Thermoelementen (36, 37) vorausgeht, von denen sich eines in einer ersten Position ($S_1$) des Bauwerks aus Beton (S) in der Nähe zu der genannten ersten Elektrode (2) und/oder der genannten mindestens einen zweiten Elektrode (3) befindet und das andere sich an einer zweiten Position ($S_2$) im Kontakt zum Bauwerk aus Beton (S) in der Nähe zur genannten Gegenelektrode (15) befindet, wobei ein Schritt i) hinsichtlich der Ermittlung der jeweiligen Istwerte der Temperatur ($T_1$, $T_2$) durch die genannten Thermoelemente (36, 37) vorgesehen wird, der genannte Schritt g) zur Berechnung auf Grundlage der genannten gemessenen elektrischen Spannungen ($V_1$, $V_2$) und der genannten Istwerten der Temperaturen ($T_1$, $T_2$) ausgeführt wird, die dank der genannten Thermoelemente (36, 37) gemessen werden, wobei dem genannten Schritt f) zur Messung ein Schritt j) zum Füllen der Aufnahmeöffnung (18) für die Gegenelektrode (15) mit einer Reinigungsflüssigkeit (P) und ein Schritt k) zum Füllen der Aufnahmeöffnung (18) für die Gegenelektrode (15) mit einer leitfähigen Flüssigkeit (C) vorausgeht.

7. Ein Verfahren gemäß Patentanspruch 6, **dadurch gekennzeichnet, dass** der genannte Schritt g) zur Berechnung der Konzentration der Chlorid-Ionen und des pH-Wertes unter Verwendung eines dazu dienenden Algorithmus entsprechend der Nernst-Gleichung ausgeführt wird.

8. Ein Verfahren gemäß Patentanspruch 6, **dadurch gekennzeichnet, dass** der genannte Schritt g) zur Berechnung in bestimmten Zeitabständen wiederholt wird, um eine Zeitkurve der Konzentration der Chlorid-Ionen und des pH-Wertes in dem Bauwerk aus Beton (S) zu erstellen.

**Revendications**

1. Un système de détection et de surveillance de la concentration en ions chlorure et du pH dans une structure en béton (S), comprenant :

- au moins une première électrode (2) pour la détection d'ions chlorure, destinée à être intégrée dans un trou de logement respectif (16) de la structure complète (S) ;
- au moins une seconde électrode (3) pour la détection du pH, destinée à être intégrée dans un trou de logement respectif (17) de la structure en béton (S) ;
- une contre-électrode (15), destinée à être insérée dans un trou de logement respectif (18) de la structure en béton (S) en contact avec celle-ci ;
- un moyen de microprocesseur (28) comprenant une unité de mesure (29) pour la mesure de tensions électriques ($V_1$, $V_2$) entre ladite contre-électrode (15) et ladite au moins une première électrode (2) ou ladite au moins une seconde électrode (3), et une unité de traitement (32) ;

caractérisé en ce que le système comprend au moins deux thermocouples (36, 37), dont l'un est apte à se trouver au niveau d'un premier emplacement ($S_1$) de la structure en béton (S) à proximité de ladite au moins une première électrode (2) et/ou de ladite au moins une seconde électrode (3) et l'autre est apte à se trouver au niveau d'un second emplacement ($S_2$) en contact avec la structure en béton (S) à proximité de ladite contre-électrode (15) pour mesurer des températures instantanées respectives ($T_1$, $T_2$), ladite unité de traitement (32) étant reliée auxdits thermocouples (36, 37) et étant apte à calculer la concentration en ions chlorure et le pH à partir des valeurs mesurés desdits tensions électriques ($V_1$, $V_2$) et desdites valeurs de température instantanée ($T_1$, $T_2$),
et en ce que le système comprend également un circuit de nettoyage (24) apte à fournir un liquide de nettoyage (P) et un circuit d'alimentation (25) apte à alimenter un liquide conducteur (C) au trou de logement (18) pour ladite contre-électrode (15).

2. Un système selon la revendication 1, **caractérisé en ce que** ladite au moins une première électrode (2) possède une première partie active Ag/AgCl (4) apte à se trouver à l'intérieur de la structure en béton (S) et une première partie conductrice en cuivre (5) apte à faire saillie de la structure en béton (S).

3. Un système selon la revendication 1, **caractérisé en ce que** ladite au moins une seconde électrode (3) possède une seconde partie active $Ir/IrO_2$ (11) apte à se trouver à l'intérieur de la structure en béton (S) et une seconde partie conductrice en cuivre (12) apte à faire saillie de la structure en béton (S).

4. Un système selon la revendication 1, **caractérisé en ce que** ladite unité de traitement (32) comprend une zone de stockage (33), dans laquelle est installé un programme informatique (A) comprenant une série d'instructions pour le calcul de la concentration d'ions chlorure et du pH à l'aide d'un algorithme approprié correspondant à la formule de Nernst.

5. Un système selon la revendication 1, **caractérisé en ce que** chacun desdits circuits (24, 25) comprend une pompe de dosage respective (26, 27), ledit moyen de microprocesseur (28) comprenant une unité de commande électronique (34) reliée à chacune des pompes de dosage (26, 27) pour commander l'alimentation dudit liquide de nettoyage (P) et/ou dudit liquide conducteur (C) dans ledit trou de logement (18) pour ladite contre-électrode (15).

6. Procédé de détection et de surveillance de la concentration de chlorure et du pH dans une structure en béton à l'aide du système selon l'une des revendications précédentes, ledit procédé comprenant les étapes consistant à:

a) prévoir au moins une première électrode (2) pour la détection d'ions chlorure dans la structure en béton (S) et l'insérer dans un trou de logement respectif (16) de la structure en béton (S);
b) prévoir au moins une seconde électrode (3) pour la détection du pH dans la structure en béton (S) et l'insérer dans un trou de logement respectif (17) de la structure en béton (S);
c) incorporer lesdites au moins une première électrode (2) et une seconde électrode (3) dans la structure en béton (S) avant ou après la coulée de béton (G);
d) prévoir une contre-électrode (15) ;
e) positionner ladite contre-électrode (15) en contact avec la structure (S) à proximité de ladite au moins une première électrode (2) et de ladite au moins une seconde électrode (3) et l'insérer dans un trou de logement respectif (18) de la structure en béton (S);
f) mesurer des tensions électriques ($V_1$, $V_2$) entre ladite contre-électrode (15) et ladite au moins une première électrode (2) et ladite au moins une seconde électrode (3);
g) calculer la concentration d'ions chlorure et les valeurs de pH par le moyen de microprocesseur (28);

caractérisé en ce que ladite étape g) de calcul est précédée par une étape consistant à

h) positionner au moins deux thermocouples (36, 37), dont l'un se trouve au niveau d'un premier emplacement ($S_1$) de la structure en béton (S) à proximité de ladite au moins une première électrode (2) et/ou de ladite au moins une seconde électrode (3) et l'autre se trouve au niveau d'un second emplacement ($S_2$) en contact avec la structure en béton (S) à proximité de ladite contre-électrode (15), une étape étant prévue, consistant à
i) détecter des températures instantanées respectives ($T_1$, $T_2$) à l'aide desdits thermocouples (36, 37), ladite étape g) de calcul étant effectuée sur la base desdites tensions électriques mesurées ($V_1$, $V_2$) et desdites valeurs de température instantanée ($T_1$, $T_2$) mesurées par lesdits thermocouples (36, 37), ladite étape de mesure f) étant précédée d'une étape consistant à
j) remplir le trou de logement (18) pour la contre-

**EP 3 114 477 B1**

électrode (15) avec un liquide de nettoyage (P) et une étape k) consistant à remplir le trou de logement (18) pour la liquide conductrice (15) avec un liquide conducteur (C).

7. Procédé selon la revendication 6, **caractérisé en ce que** ladite étape g) de calcul de la concentration d'ions chlorure et du pH est effectué par un algorithme correspondant à l'équation de Nernst.

8. Procédé selon la revendication 6, **caractérisé en ce que** ladite étape g) de calcul est répétée à des intervalles de temps prédéterminés pour déterminer une courbe de temps de la concentration d'ions chlorure et du pH dans la structure en béton (S).

**FIG. 1**

**FIG. 2**

**FIG. 3**

**FIG. 4**

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008179179 A1

- WO 2007109560 A1

**Non-patent literature cited in the description**

- **RONG-GUI DU et al.** In Situ Measurement of CI - Concentrations and pH at the Reinforcing Steel/Concrete Interface by Combination Sensors. *ANALYTICAL CHEMISTRY,* 01 May 2006, vol. 78 (9), ISSN 0003-2700, 3179-3185